# EUROPEAN PATENT SPECIFICATION

(11) **EP 4 054 449 B1**
(45) Date of publication and mention of the grant of the patent: **04.12.2024**
(21) Application number: 20804346.3
(22) Date of filing: 03.11.2020
(51) Int. Cl.: A61B 17/32, A61B 17/16

(54) **FLEXIBLE DRIVESHAFT WITH CHANNELS CONFIGURED TO COLLAPSE UNDER ROTATIONAL LOAD**
BIEGSAME ANTRIEBSWELLE MIT UNTER ROTATIONSLAST ZUSAMMENFALLENDEN KANÄLEN
ARBRE D'ENTRAÎNEMENT FLEXIBLE AVEC CANAUX CONFIGURÉS POUR S'AFFAISSER SOUS CHARGE ROTATIVE

(30) Priority: 05.11.2019 US 201962930820 P
(43) Date of publication of application: 14.09.2022
(73) Proprietor: Stryker European Operations Limited, Carrigtwohill, Co. Cork T45 HX08 (IE)
(72) Inventor: NUNAN, Gerard, Ballincollig, Co. Cork, P31 F761 (IE)
(74) Representative: Röthinger, Rainer
(86) International application number: PCT/IB2020/060309
(87) International publication number: WO 2021/090165

(56) References cited:
- EP-A2- 1 720 464
- US-A- 5 322 505
- US-B2- 9 186 157

## Description

### PRIORITY CLAIM

This application claims priority to and the benefit of U.S. Provisional Patent Application No. 62/930,820, filed on November 5, 2019.

### BACKGROUND

It is known that medical practitioners have found it useful to use surgical instruments to assist in the performance of surgical procedures. A surgical instrument is designed to be applied to a surgical site on the patient. The practitioner is able to position the surgical instrument at the site on the patient at which the instrument is to perform a medical or surgical procedure. Endoscopic surgical procedures are routinely performed in order to accomplish various surgical tasks. In an endoscopic surgical procedure, small incisions, called portals, are made in the patient. An endoscope, which is a device that allows medical personnel to view the surgical site, is inserted in one of the portals. Surgical instruments used to perform specific surgical tasks are inserted into other portals. The surgeon views the surgical site through the endoscope to determine how to manipulate the surgical instruments in order to accomplish the surgical procedure. An advantage of performing endoscopic surgery is that, since the portions of the body that are cut open are minimized, the portions of the body that need to heal after surgery are likewise reduced. Moreover, during an endoscopic surgical procedure, only relatively small portions of the patient's internal organs and tissue are exposed to the open environment. This minimal opening of the patient's body lessens the extent to which a patient's organs and tissue are open to infection

Many tube devices have been developed for use in surgical procedures. They are valuable because they facilitate reduced incision size, improved access and visibility, while enhancing surgical outcome and quicker recovery. Some are surgical cutting tools that use a burr or shaver to remove bone or tissue. The surgical cutting tools include a driveshaft that rotates at a high rate of speed, such as 50,000 revolutions per minute. The driveshaft is enclosed by an outer tube that supports the driveshaft and protects the driveshaft from contacting tissue within the body. The close fit between the driveshaft and the outer tube generates a large amount of friction and heat. A surgical instrument that overcomes these challenges is desired.

Surgical instruments are known from US 5 322 505 A, US 9 186 157 B2 and EP 1 720 464 A2.

### SUMMARY

The invention provides a surgical cutting tool according to claim 1. The dependent claims refer to developments of the invention.
A first aspect of the present disclosure is directed to a surgical cutting tool is configured for use with a handpiece having a motor. The surgical cutting tool includes an outer tube extending longitudinally between proximal and distal ends. The outer tube includes an inner surface defining a lumen therethrough. The surgical cutting tool further includes a flexible driveshaft including an inner tube rotatably disposed in the lumen and extending in a longitudinal direction past the distal end to a cutting end outside of the lumen. The driveshaft further includes at least two torsion sections spaced from one another longitudinally along the inner tube. The at least two torsion sections each have at least one channel extending through the inner tube. The driveshaft further includes a bearing section disposed longitudinally between the torsion sections. The channels are configured to collapse under rotational load in response to transmission of torque as the driveshaft rotates such that the torsion sections are spaced apart from the inner surface of the outer tube to a greater extent than the bearing section for reducing friction between the inner tube and the outer tube.

In certain implementations, the inner tube includes a pair of channel walls individually corresponding to and defining each of the at least one channel. The pair of channel walls may oppose and face toward one another to define a width of the channel therebetween. The width may be at least 15 microns. The pair of channel walls may extend substantially parallel to one another. The pair of channel walls may be configured to draw toward one another and collapse the channel when under rotational load in response to transmission of torque with rotation of the driveshaft. The outer diameter of the torsion sections is configured to reduce at least five percent when the channels collapse. The outer diameters of the torsion and bearing sections may be substantially equal when the driveshaft is stationary.

In certain implementations, the at least one channel includes a plurality of channels in each torsion section and evenly spaced from one another. The plurality of channels may have a uniform size and shape. The plurality of channels may extend in a helical configuration longitudinally along and about the inner tube. For example, the plurality of channels may extend about the inner tube less than 180 degrees or less than 360 degrees. The plurality of channels in the helical configuration may extend at an angle at or between 30 and 60 degrees from an axis orthogonal to the longitudinal direction of the inner tube.

In certain implementations, the outer tube and the lumen are disposed in a non-linear configuration between the ends, and the driveshaft may be correspondingly disposed in the non-linear configuration within the lumen. A length of the torsion sections may have a ratio of at least 2:1 relative to a length of the bearing section.

A second aspect of the present disclosure is directed to a flexible driveshaft is configured for use with a surgical cutting tool. The driveshaft is configured to be rotatably disposed in a lumen of an outer tube. The driveshaft includes an inner tube extending in a longitudinal direction to a cutting end, and a torsion section having an outer diameter and a plurality of channels extending through the inner tube. The plurality of channels extends in a helical configuration longitudinally along and about the inner tube in a first rotational direction. The driveshaft is configured to rotate in a second rotational direction, opposite the first rotational direction. The channels are configured to collapse under rotational load in response to transmission of torque as the driveshaft rotates in the second rotational direction such that the outer diameter of the torsion section is reduced.

In certain implementations, the flexible driveshaft according to the second aspect of the present disclosure may be included on the surgical cutting tool of the first aspect, the third aspect, and/or the fourth aspect, and optionally, any of their corresponding implementations.

A third aspect of the present disclosure is directed to a surgical cutting tool is configured for use with a handpiece having a motor. The surgical cutting tool includes an outer tube extending longitudinally between proximal and distal ends. The outer tube includes an inner surface defining a lumen therethrough. The surgical cutting tool further includes a flexible driveshaft including an inner tube rotatably disposed in the lumen and extending in a longitudinal direction past the distal end to a cutting end outside of the lumen. The driveshaft includes pairs of channel walls individually corresponding to and defining channels. The pairs of channel walls may oppose and face toward one another to define a width of the channels therebetween. The width may be at least 15 microns. The channels are configured to collapse under rotational load in response to transmission of torque.

A fourth aspect of the present disclosure is directed to a surgical cutting tool is configured for use with a handpiece having a motor. The surgical cutting tool includes an outer tube extending longitudinally between proximal and distal ends. The outer tube includes an inner surface defining a lumen therethrough. The surgical cutting tool further includes a flexible driveshaft including an inner tube rotatably disposed in the lumen and extending in a longitudinal direction past the distal end to a cutting end outside of the lumen, wherein a portion of the flexible driveshaft is configured to collapse under rotational load in response to transmission of torque.

A method of operating a surgical cutting tool for use with a handpiece having a motor is further disclosed herein. The surgical cutting tool includes an outer tube extending longitudinally between proximal and distal ends and including an inner surface defining a lumen therethrough. The surgical cutting tool further includes a flexible driveshaft including an inner tube rotatably disposed in the lumen and extending in a longitudinal direction past the distal end to a cutting end outside of the lumen. The driveshaft further includes at least two torsion sections spaced from one another longitudinally along the inner tube and each a having at least one channel extending through the inner tube. The driveshaft further includes a bearing section disposed longitudinally between the torsion sections. The method includes the steps of rotating the driveshaft to transmit torque, applying a rotational load to the driveshaft, collapsing the channels, and spacing the torsion sections from the inner surface of the outer tube to a greater extent than the bearing section for reducing friction between the inner tube and the outer tube.

### BRIEF DESCRIPTION OF THE DRAWINGS

Figure 1 is a perspective view of a surgical cutting tool coupled to a handpiece.
Figure 2 is a cross-sectional view of the surgical cutting tool taken along line 2-2 in Figure 1, showing a flexible drive shaft and an outer tube.
Figure 3 is a cross-sectional view of the surgical cutting tool taken along line 3-3 in Figure 1, showing a flexible drive shaft and an outer tube.
Figure 4 is a perspective view of a portion of the flexible driveshaft showing bearing sections and torsion sections having channels.
Figure 5 is an elevational view of a portion of the flexible driveshaft showing lengths and outer diameters of the bearing and torsion sections.
Figure 6 is an elevational view of a portion of the flexible driveshaft showing outer diameters of the bearing and torsion sections when the channels of the torsion sections collapse under a rotational load.
Figure 7 is a partial, cross-sectional view of flexible driveshaft and the outer tube, with the outer tube in a non-linear configuration and the channels collapsed under the rotational load.
Figure 8 is a partial, cross-sectional view of flexible driveshaft and the outer tube, with the outer tube in a linear configuration and the channels collapsed under the rotational load.
Figure 9 is a schematic plan view of the flexible driveshaft split and unwrapped along a longitudinal direction.
Figure 10 is a partial, cross-sectional view of flexible driveshaft and the outer tube, with the channels collapsed under a first rotational load.
Figure 11 is a partial, cross-sectional view of flexible driveshaft and the outer tube, with the channels collapsed under a second rotational load.
Figure 12 is a cross-sectional view of a portion of the flexible driveshaft taken along line 12-12 in Figure 9.

### DETAILED DESCRIPTION

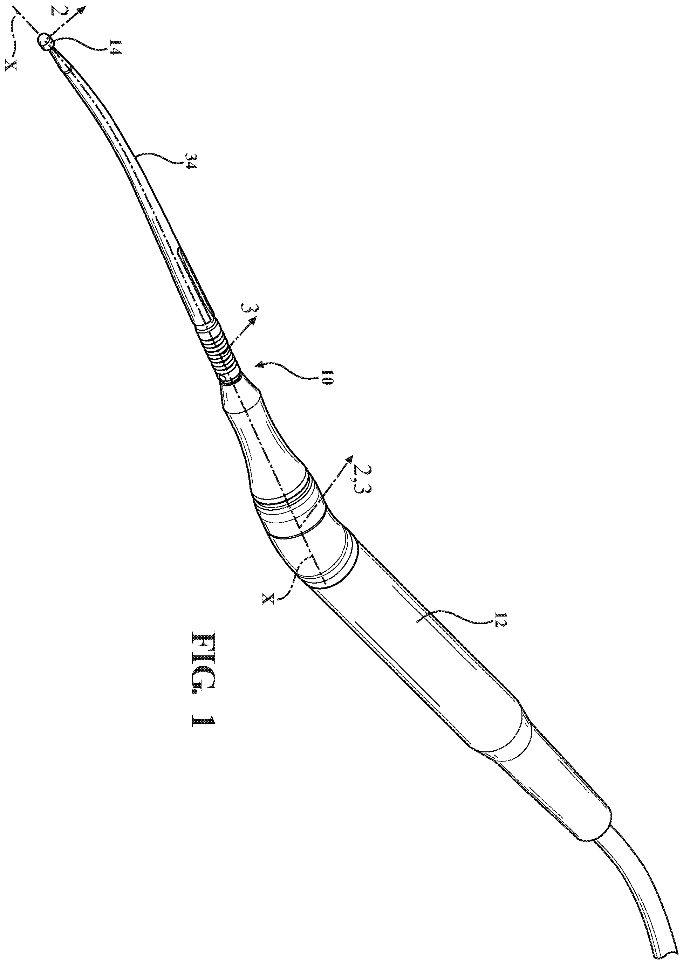

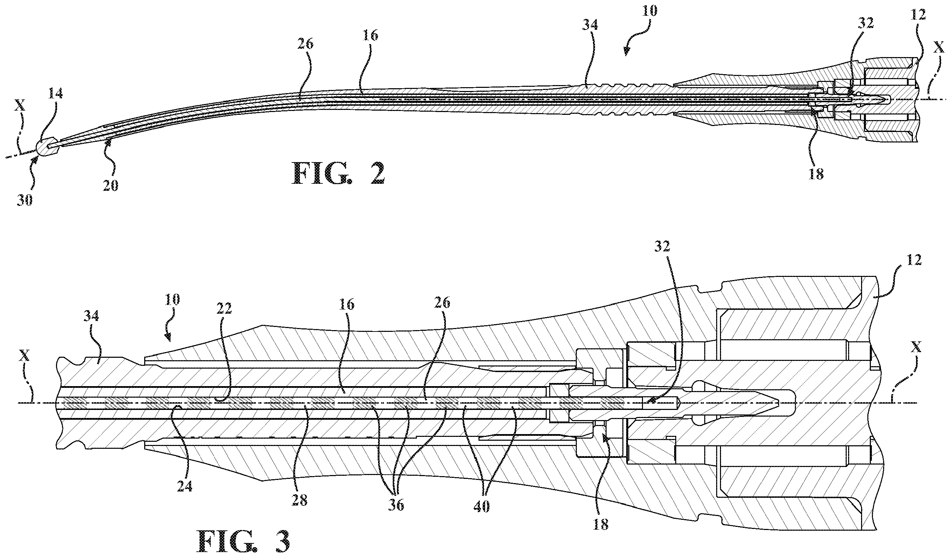

Referring to Figure 1, a surgical cutting tool is shown at 10 for use with a handpiece 12 having a motor. The surgical cutting tool 10 as shown in the Figures and described below is used in a medical procedure for a patient (not shown). The surgical cutting tool 10 may comprise an attachment 14, such as burr (shown in the Figures 1 and 2) or a shaver. The motor of the handpiece 12 drives the surgical cutting tool 10 at a high rate of speed. Exemplary uses of the attachment 14 include abrading and resecting bone and tissue, such as during endoscopic sinus surgery. However, the surgical cutting tool 10 may also be adapted for other medical procedures, including but not limited to, spinal, neuro, and endoscopic applications. It should be appreciated that the surgical cutting tool 10 may be operated by a user (not shown) such as a surgeon.

As shown in Figures 2 and 3, the surgical cutting tool 10 includes an outer tube 16 extending longitudinally between proximal and distal ends 18, 20 and including an inner surface 22 defining a lumen 24 therethrough. The surgical cutting tool 10 further includes a flexible driveshaft 26 including an inner tube 28 rotatably disposed in the lumen 24. The driveshaft 26 extends in a longitudinal direction X past the distal end 20 to a cutting end 30 outside of the lumen 24. The aforementioned attachment 14 may be coupled to the cutting end 30 of the driveshaft 26. Although the attachment 14 is shown in the Figures as a burr, any suitable attachment may be coupled to the cutting end 30 of the driveshaft 26. The driveshaft 26 further includes a motor end 32 opposite the cutting end 30. The driveshaft 26 is coupled to the motor of the handpiece 12 at the motor end 32, with the motor configured to selectively rotate the driveshaft 26. The motor transfers torque to the driveshaft 26, which rotates the attachment 14 disposed at the cutting end 30 of the driveshaft 26. The motor may rotate the driveshaft 26 and the attachment 14 at speeds greater than 50,000 revolutions per minute. The high-speed rotation and torque transfer from the motor to the attachment 14 allows the surgical cutting tool 10 to accurately and efficiently abrade or resect the bone and tissue during the surgical procedure as described above. Although the attachment 14 is configured to be rotated by the driveshaft 26 in the Figures, the attachment 14 may be configured for non-rotatable motion (such as reciprocation) in other examples not shown herein.

The outer tube 16 and the inner tube 28 may have a corresponding slip fit which allows the inner tube 28 to rotate axially within the lumen 24 while laterally retaining the inner tube 28 within the lumen 24. As such, the outer tube 16 supports the inner tube 28 and reduces wobbling of the inner tube 28 during rotation. Furthermore, the outer tube 16 protects the inner tube 28 from invasion of material (*e.g*., tissue of the body) that could inhibit the operation of the inner tube 28, such as binding the outer and inner tubes 16, 28 and slowing the rotation of the inner tube 28. The outer tube 16 also protects the patient from tissue degradation due to contact with the inner tube 28 (*i.e.,* friction and heat from the rotating inner tube 28).

The surgical cutting tool 10 may further comprise a nose tube 34 surrounding the outer tube 16 and extending longitudinally between the attachment 14 and the handpiece 12. The nose tube 34 may be tapered as shown in the Figures to ease the insertion of the surgical cutting tool 10 into the body. The outer tube 16 may be fixed to the nose tube 34, with the nose tube 34 further supporting the outer tube 16. The nose tube 34 may further cover and protect the rotatable inner tube 28 from the body of the patient and vice-versa. Although the outer tube 16 and the nose tube 34 are shown as separate components, the outer tube 16 and the nose tube 34 may be an integral, unitary component in other examples not shown herein. As such, in other examples the outer tube 16 is the outer most component that interfaces the body of the patient.

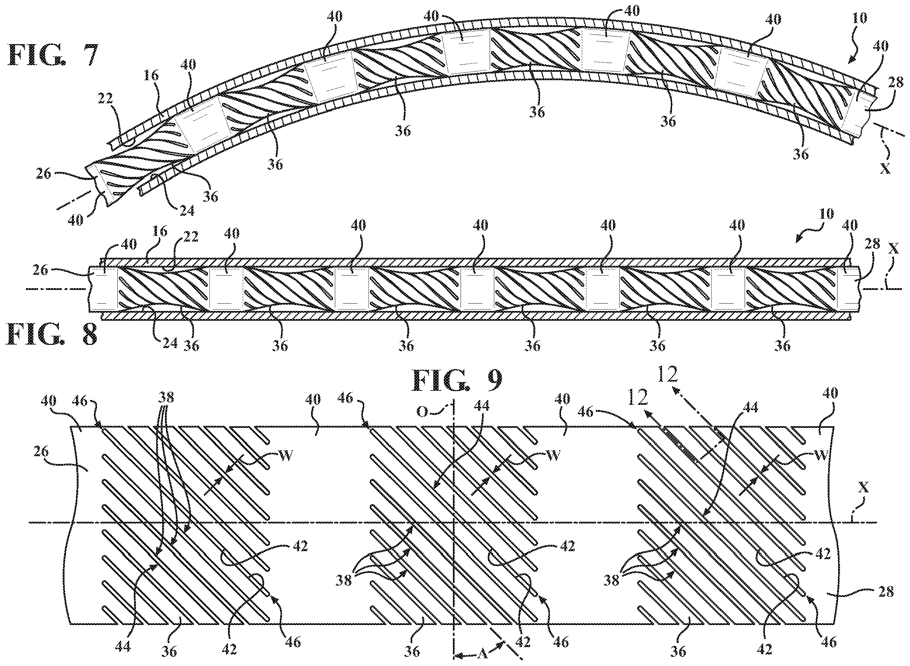

As shown in Figure 8, the outer tube 16 and the lumen 24 may be disposed in a linear configuration between the ends, and the driveshaft 26 may be correspondingly disposed in the linear configuration within the lumen 24. On the other hand, the outer tube 16 and the lumen 24 may be disposed in a non-linear configuration between the ends, and the driveshaft 26 may be correspondingly disposed in the non-linear configuration within the lumen 24, as shown in Figure 7. Moreover, the outer tube 16 and the driveshaft 26 may vary between linear and non-linear configurations at different portions of the surgical cutting tool 10. For example, the outer tube 16 and lumen 24 may be disposed in the linear configuration at the proximal end 18 of the outer tube 16 and in the non-linear configuration at the distal end 20 of the outer tube 16, as shown in Figure 2. Transnasal applications of the surgical cutting tool 10 will often employ such a bend at the distal end 20. On the other hand, spinal applications of the surgical cutting tool 10 will often employ a bend at the proximal end 18.

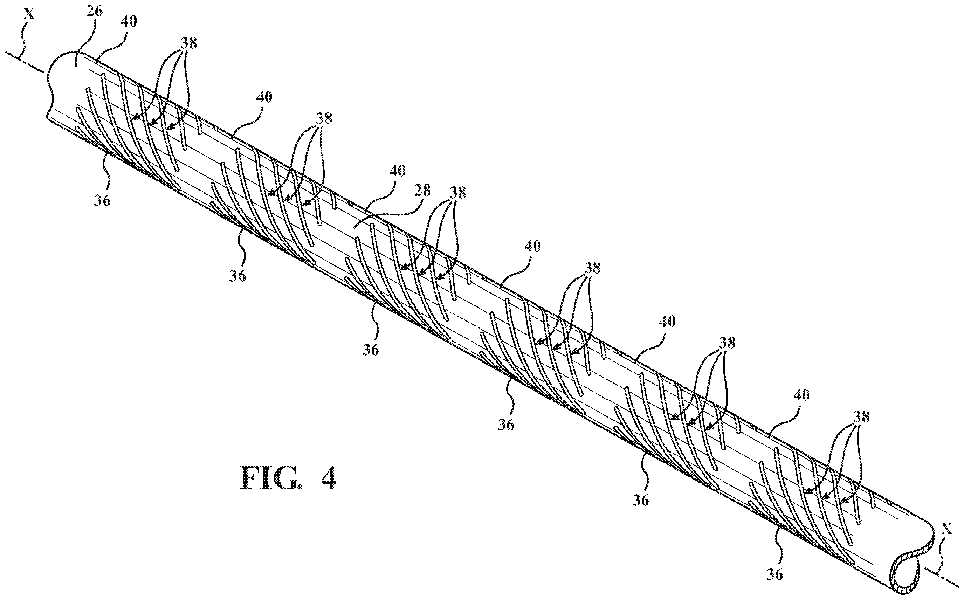

As shown in Figures 4-6, the driveshaft 26 may further comprise at least two torsion sections 36 spaced from one another longitudinally along the inner tube 28 and each having at least one channel 38 extending through the inner tube 28, and a bearing section 40 disposed longitudinally between the torsion sections 36. In the example shown in the Figures, the at least one channel 38 includes a plurality of channels 38 evenly spaced from one another. More specifically, each torsion section 36 has a plurality of channels 38 extending through the inner tube 28. In the example shown in the Figures, each torsion section 36 includes eight channels 38. The number of channels 38 may vary depending on the diameter of the inner tube 28. For exemplary purposes, the number of channels 38 may be within the range of 4 to 40, within the range of 5 to 30, or within the range of 6 to 20. However, the torsion sections 36 may have any number of channels 38, and the number of channels 38 may be based on imparting the desired flexural and bearing properties to be described in detail.

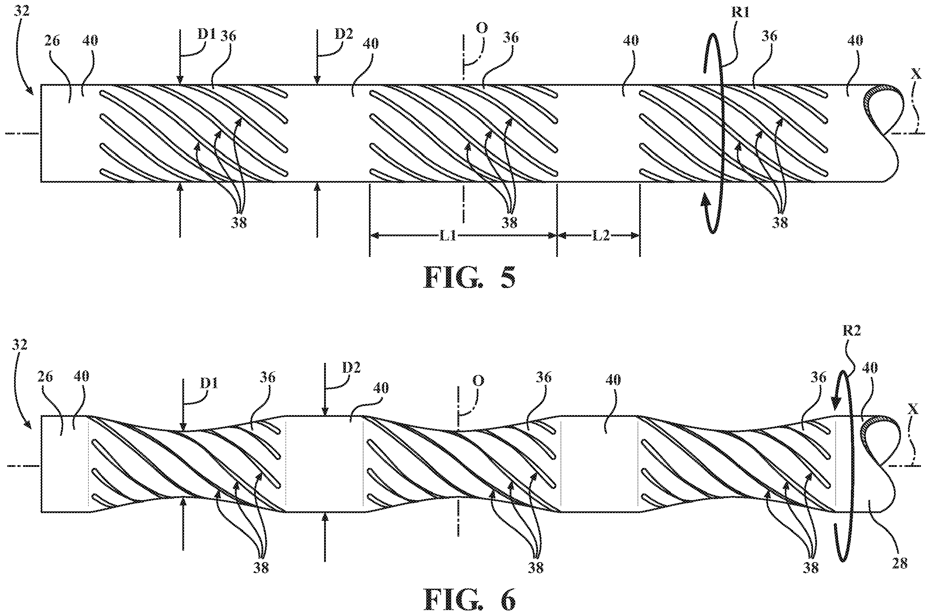

Moreover, the at least two torsion sections 36 and the bearing section 40 may be further defined as a plurality of torsion sections 36 and a plurality of bearing sections 40, with the torsion sections 36 and bearing sections 40 individually staggered along driveshaft 26 (*i.e.,* a bearing section 40, followed by a torsion section 36, followed by a bearing section 40, followed by a torsion section 36, *etc.*)*.* As shown in Figure 5, each of the torsion and bearing sections 40 may have a length L1, L2 along the longitudinal direction X of the inner tube 28, with the length L1 of the torsion sections 36 having a ratio of at least 2:1 relative to the length L2 of the bearing section 40. For exemplary purposes, the length L1 of the torsion sections 36 may have a ratio relative to the length L2 of the bearing section 40 ranging from 2:1 to 4:1. In one non-limiting example, the torsion sections 36 have a length L1 of 3mm and the bearing sections 40 have a length L2 of 1 mm. In general, the greater the ratio between the torsion and bearing sections 36, 40, the greater the flexibility between of the driveshaft 26. The greater the flexibility, the more the driveshaft 26 is able to bend to accommodate non-linear configurations of the outer tube 16. However, the torsion and bearing sections 40 may have any suitable length L1, L2 and ratio. Furthermore, although the torsion sections 36 are shown in the Figures to have a uniform length L1 and the bearing sections 40 are shown in the Figures to have a uniform length L2, each of the lengths L1, L2 may vary. Therefore, the ratio described above may apply to one torsion section 36 and one bearing section 40 that are adjacent to one another. Furthermore, because the lengths L1, L2 may vary, the ratios between the adjacent torsion and bearing sections 36, 40 may vary as well.

The bearing and torsion sections 36, 40 may be integrally formed of a single material. More specifically, the driveshaft 26 may be formed of the single material. In one example, the single material is stainless steel. However, any material capable of transmitting torque may be utilized.

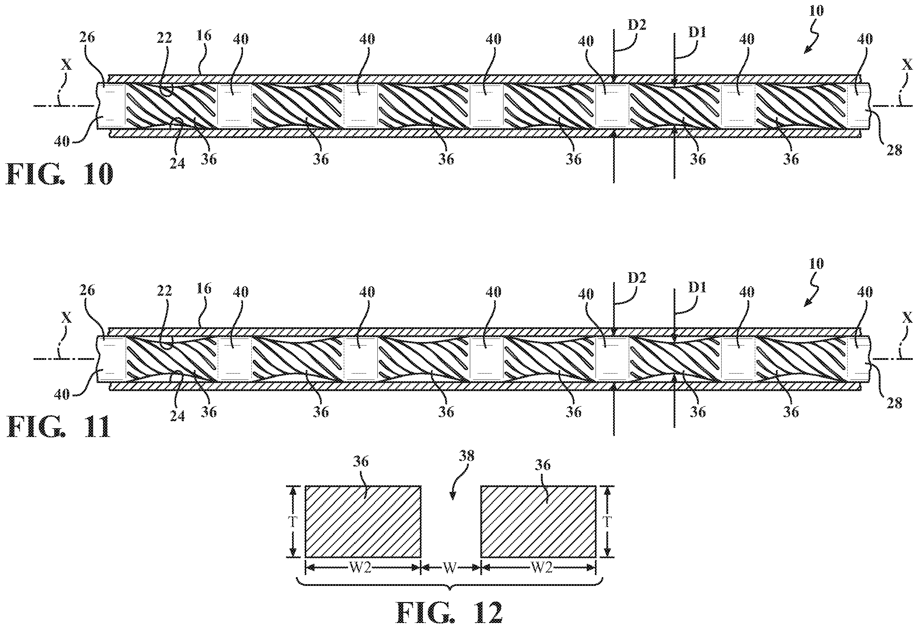

The inner tube 28 may have an interior surface defining an inner diameter and an exterior surface defining an outer diameter. For exemplary purposes, the outer diameter may range from 0.5 mm to 5 mm. However, the outer diameter may be any suitable measurement depending on the application of the driveshaft 26. Moreover, the interior and exterior surfaces may define a cross-sectional thickness T of the inner tube 28, therebetween, as shown in Figure 12. In one non-limiting example, the inner tube 28 is comprised of a 16.5 gauge hypotube. However, any suitable tubing may be used.

Turning to Figure 2, because the driveshaft 26 is typically comprised of stainless steel, the attachment 14 may fixed to the cutting end 30 of the driveshaft 26 by welding. Moreover, when the driveshaft 26 is comprised of the hypotube, the ends of the hypotube *(i.e.,* the cutting and motor ends 30, 32) provide sockets that facilitate an improved welding interface. Known devices may utilize a cable the driveshaft with the attachment welded to strands of the cable. In such an arrangement, the strands are prone to fraying at ends, and welding involves attaching each of the strands of the cable to the attachment. The welding process may undesirably melt the individual strands. Utilizing the inner tube 28 possibly of unitary construction and having the aforementioned socket may require welding only two components together with greater contact area between the two for the improved welding interface.

In one example, the channels 38 are formed by laser cutting the inner tube 28. However, the channels 38 may be formed using other suitable machining/forming processes, including electric discharge machining and 3D printing.

As shown in Figures 7 and 8, the channels 38 are configured to collapse under rotational load in response to transmission of torque as the driveshaft 26 rotates such that the torsion sections 36 are spaced apart from the inner surface 22 of the outer tube 16 to a greater extent than the bearing section 40 for reducing friction between the inner tube 28 and the outer tube 16. As shown in the schematic view of Figure 9, the inner tube 28 may comprise a pair of channel walls 42 individually corresponding to and defining each of the at least one channel 38. More specifically, each of the channels 38 includes a pair of channel walls 42. The pair of channel walls 42 may oppose and face toward one another to define a width W of the channel 38 therebetween. Said differently, the pair of channel walls 42 are spaced from one another and thus define the channel 38.

The pair of channel walls 42 may extend substantially parallel to one another. Moreover, the plurality of channels 38 may have a uniform size and shape. Said differently, the channels 38 are shown in the Figures to have a uniform spacing between the pair of channel walls 42 along the entirety of the channel walls 42 (*i.e.,* the width W of the channel 38 may be consistent along the length of the channel 38). Moreover, the width W of each of the channels 38 shown is shown in the Figures to be substantially equally. As shown in the schematic view of Figure 9, the channels 38 in the helical configuration extend linearly along the inner tube 28. However, the channels 38 may be curved or any other suitable shape.

However, in other examples not shown herein the width W of each of the channels 38 may vary along the length of the channel 38, as well as vary between different channels 38. The width W between the pair of channel walls 42 may be at or between 15-40 microns. The greater the width W, the more that the torsion sections 36 are able to space from the outer tube 16 under rotational load. However, the width W between the pair of channel walls 42 may be any suitable value that permits the collapse of the channel 38 when under rotational load.

As shown in Figure 12, portions of the inner tube 28 between adjacent channels 38 may have a second width W2 (*i.e.,* the distance between the adjacent channels 38). The second width W2 of the inner tube 28 may be greater than the aforementioned cross-sectional thickness T of the inner tube 28 to facilitate the collapse of the channels 38 in the torsions sections 36 while ensuring localized stresses on the torsion sections 36 are managed. As one non-limiting example, the second width W2 and the cross-sectional thickness T have a ratio of at least 2:1. However, any suitable sizing of the second width W2 and the cross-sectional thickness T may be used. Moreover, the second width W2 between adjacent channels 38 and the cross-sectional thickness T of the inner tube may vary around the inner tube 28.

As shown in Figures 7 and 8, the pair of channel walls 42 are configured to draw toward one another and collapse the channel 38 when under rotational load in response to transmission of torque as the driveshaft 26 rotates. More specifically, the localized reduction of material in the torsion section 36 at the channels 38 locally weakens the torsion section 36 and facilitates deflection of the driveshaft 26 at the torsion section 36. The rotational load occurs when the rotating driveshaft 26 experiences an opposing torque. Most commonly, the opposing torque is exerted by the material that the attachment 14 engages to remove (*i.e.,* bone and tissue). The rotating attachment 14 engages the stationary material, which exerts a torque on the attachment 14 which opposes the rotation of the attachment 14. The opposing torque is transmitted to the driveshaft 26. The channels 38 collapse causing the torsion section 36 to deflect under the rotational load. The collapse of the channel 38 causes the opposing pair of channel walls 42 to draw toward one another. The channel 38 may collapse until the pair of channel walls 42 contact. The contact between the pair of channel walls 42 may restore the torsional rigidity to the torsion sections 36 (*i.e.,* the lack of the channel 38 does not promote further deflection in the torsion sections 36 and therefore strengthens the torsional rigidity of the torsion sections 36). The torsional rigidity of the torsion region may become substantially equal to the bearing sections 40 of the driveshaft 26. However, the pair of channel walls 42 need not contact to continue to transmit rotational motion to the attachment 14. If the rotational load exerted through the contact of the attachment 14 with the material (*i.e.,* the tissue or bone) is of a small enough value, the torsion sections 36 may have sufficient rigidity even with the channel walls 42 spaced from one another to continue to rotate the attachment 14.

The collapse of the channels 38 under rotational load causes the torsion sections 36 to be spaced apart from the inner surface 22 of the outer tube 16 to a greater extent than the bearing sections 40 for reducing friction between the inner tube 28 and the outer tube 16. More specifically, each of the torsion and bearing sections 40 may have an outer diameter D1, D2, as shown in Figures 5 and 6. The outer diameters D1, D2 of the torsion and bearing sections 40 may be substantially equal when the driveshaft 26 is stationary. However, the outer diameter D1 of the torsion sections 36 may be configured to reduce at least five percent when the channels 38 collapse. More specifically, the outer diameter D1 of the torsion sections 36 may be configured to reduce at or between five and ten percent when the channels 38 collapse. The reduction in diameter causes the torsion sections 36 to be spaced from the outer tube 16 when under rotational load. Therefore while the entire driveshaft 26 engages the outer tube 16 when the driveshaft 26 is stationary, only portions of the driveshaft 26 engage the outer tube 16 when the driveshaft 26 is under rotational load (namely, the bearing sections 40). As such, the surface area of the driveshaft 26 in contact with the outer tube 16 reduces under rotational load, which reduces friction and heat between the driveshaft 26 and outer tube 16 during operation of the surgical cutting tool 10.

To facilitate the reduction in the outer diameter D1 of the torsion sections 36 when the channels 38 collapse, the channels 38 may extend in a helical configuration longitudinally along and about the inner tube 28. More specifically, the channels 38 may extend in the helical configuration longitudinally along and about the inner tube 28 in a first rotational direction R1. The first rotational direction R1 of the channels 38 refers to the direction around the driveshaft 26 that the channels 38 progress from the motor end 32 toward the cutting end 30. The first rotational direction R1 is illustrated in Figure 5. In Figure 5, the motor end 32 is disposed on the left side of the page, with the channels 38 twisting about the longitudinal direction X in a clockwise direction as the channels 38 progress away from the motor end 32. The driveshaft 26 is configured to rotate in a second rotational direction (shown at R2 in Figure 6), opposite the first rotational direction R1. The channels 38 are configured to collapse under rotational load in response to transmission of torque as the driveshaft 26 rotates in the second rotational direction R2 such that the outer diameter D1 of the torsion section 36 is reduced. More specifically, when the driveshaft 26 rotates in the first rotational direction R1, the rotational load (*i.e.,* the opposing torque) that is exerted on the driveshaft 26 acts in the first rotational direction R1. As such, the opposing channels walls 42 are pushed toward one another causing the channels 38 to collapse. The channels 38 collapse causing the torsion sections 36 to deflect under the rotational load.

The collapse of the channels 38 facilitates the reduction in the outer diameter D1 of the torsion sections 36 because the circumference of the driveshaft 26 in the torsion sections 36 reduces when the channels 38 collapse. More specifically, as is commonly known in geometry, the circumference of a circle is equal to the diameter of the circle multiplied by pi (*i.e.,* C = πd). Therefore, a reduction in the circumference of a circle would proportionally reduce the diameter of the circle. In the example shown in the Figures, the channels 38 occupy and define a portion of the circumference of the driveshaft 26 when no rotational load is exerted on the driveshaft 26. When the channels 38 collapse under the rotational load, the circumference of the driveshaft 26 reduces, which correspondingly reduces the outer diameter D1 of the torsion sections 36.

As shown in Figure 9, the channels 38 in the helical configuration may extend at an angle A at or between 30 and 60 degrees from an axis O orthogonal to the longitudinal direction X of the inner tube 28. The angle A of the channels 38 in the helical configuration may dictate how the driveshaft 26 reacts when the channels 38 collapse. More specifically, the angle A of the channel 38 may affect the flexibility of the torsion sections 36 and the reduction of the outer diameter D1 of the torsion sections 36 when under rotational load. For example, increasing the angle A between the channel 38 and the axis O orthogonal to the longitudinal direction X may result in torsion sections 36 becoming more flexible but may decrease the amount that the outer diameter D1 constricts when the channels 38 collapse. Conversely, decreasing the angle A between the channel 38 and the axis O orthogonal to the longitudinal direction X may result in torsion sections 36 becoming less flexible but may increase the amount that the outer diameter D1 constricts when the channels 38 collapse. As such, the angle A of the channels 38 may be configured to provide the desired flexibility and outer diameter D1 reduction of the torsion sections 36 depending on the application of the surgical cutting tool 10. For example, configurations in which the outer tube 16 and the lumen 24 are non-linear as described above may require a large angle A between the channels 38 and the axis O (such as 60 degrees) to emphasis the flexibility of the driveshaft 26 within the non-linear lumen 24. On the other hand, configurations in which the outer tube 16 and the lumen 24 are linear as described above may require a small angle A between the channels 38 and the axis O (such as 30 degrees) to emphasis the friction reduction between of the driveshaft 26 and the outer tube 16. One having skill in the art will appreciate that the channels 38 may be positioned at any suitable angle A relative to the axis O, including outside of the bounds of the exemplary range given above.

The channels 38 may extend about the inner tube 28 less than 360 degrees, as shown in Figure 9. More specifically, the channels may extend about the inner tube 28 less than 270 degrees. Even more specifically, the channels 38 may extend about the inner tube 28 less than 180 degrees. The angle that the channels 38 extend about the inner tube 28 may be dictated by the length of the channels 38 and the angle A of the channels 38 relative to the axis O (*i.e*., the helical angle described above). More specifically, the greater the angle A of the channels 38 relative to the axis O, the longer the channels 38 must be to extend the same radius about the inner tube 28. As the channels 38 increase in length, more material must be removed from the inner tube 28 to form the channels 38, which improves the flexibility of the inner tube 28 but also weakens the inner tube 28. Therefore, while exemplary angles have been given above, the radius that the channels 38 extend about the inner tube 28 may be any suitable value depending on the application of the surgical cutting tool 10.

An exemplary method, not forming part of the invention, of operating the surgical cutting tool 10 is described below and shown in Figures 10. The method includes the steps of rotating the driveshaft 26 to transmit torque and applying the rotational load to the driveshaft 26. As described above, the rotational load occurs when the rotating driveshaft 26 experiences an opposing torque. Most commonly, the opposing torque is exerted by the material that the attachment 14 engages to remove (*i.e.,* bone and tissue). The rotating attachment 14 engages the stationary material, which exerts a torque on the attachment 14 which opposes the rotation of the attachment 14. The opposing torque is transmitted to the driveshaft 26.

The method further includes the step of collapsing the channels 38. As described above, the channels 38 collapse causing the torsion section 36 to deflect under the rotational load. The collapse of the channels 38 causes the opposing pair of channel walls 42 to draw toward one another. The channels 38 may collapse until the pair of channel walls 42 contact. The contact between the pair of channel walls 42 may restore the torsional rigidity to the torsion sections 36 (*i.e.,* the lack of the channel 38 does not promote further deflection in the torsion sections 36 and therefore strengthens the torsional rigidity of the torsion sections 36). The torsional rigidity of the torsion sections 36 may become substantially equal to the bearing sections 40 of the driveshaft 26.

The method further includes spacing the torsion sections 36 from the inner surface 22 of the outer tube 16 to a greater extent than the bearing section 40 for reducing friction between the inner tube 28 and the outer tube 16, which reduces friction between the inner tube 28 and the outer tube 16. More specifically, each of the torsion and bearing sections 40 may have the outer diameter D1, D2 described above. The outer diameters D1, D2 of the torsion and bearing sections 40 may be substantially equal when the driveshaft 26 is stationary. However, the outer diameter D1 of the torsion sections 36 may be configured to reduce at least five percent when the channels 38 collapse. The reduction in diameter causes the torsion sections 36 to be spaced from the outer tube 16 when under rotational load. Therefore while the entire driveshaft 26 engages the outer tube 16 when the driveshaft 26 is stationary, only portions of the driveshaft 26 engage the outer tube 16 when the driveshaft 26 is under rotational load (namely, the bearing sections 40). As such, the surface area of the driveshaft 26 in contact with the outer tube 16 reduces under rotational load, which reduces friction and heat between the driveshaft 26 and outer tube 16 during operation of the surgical cutting tool 10.

The step of applying the rotational load to the driveshaft 26 may be further defined as applying a first rotational load to the driveshaft 26, as shown in Figure 10. The method may further comprise the steps of applying a second rotational load to the driveshaft 26 that is greater than the first rotational load, further collapsing the channels 38, and further spacing the torsion sections 36 from the inner surface 22 of the outer tube 16 to a greater extent than the bearing section 40 and the torsion sections 36 under the first rotational load for further reducing friction between the inner tube 28 and the outer tube 16, as shown in Figure 11. Said differently, the spacing of the torsion sections 36 from the inner surface 22 of the outer tube 16 is proportional to amount of rotational load exerted on the driveshaft 26. The greater the rotational load (*e.g*., the second rotational load compared to the first rotational load), the greater the spacing of the torsion sections 36 from the inner surface 22 of the outer tube 16. In other words, as will become apparent below, the driveshaft 26 works more efficiently (*i.e.,* reduced friction and heat) under higher rotational speeds and rotational loads.

More specifically, the collapse of the channels 38 begins at a center 44 of each of the channels 38 (and respectively the center 44 of the torsion sections 36) and moves outwardly toward a pair of ends 46 of each of the channels 38 (schematically shown in Figure 9). As such, greater rotational load results in a greater portion of each of the channels 38 collapsing from their respective centers 44 toward the pair of ends 46. Therefore, as more of the channels 38 collapse, more of the outer surface of the inner tube 28 in the torsion sections 36 becomes spaced from the outer tube 16, which further reduces friction between the driveshaft 26 and the outer tube 16. Accordingly, the efficiency of the driveshaft 26 increases with the increase of rotational load due to the proportional decrease in friction.

The invention has been described in an illustrative manner, and it is be understood that the terminology which has been used is intended to be in the nature of words of description rather than of limitation. It is now apparent to those skilled in the art that many modifications and variations of the present invention are possible in light of the above teachings. It is, therefore, to be understood that the invention may be practiced otherwise than as specifically described. The scope of the invention is defined by the claims.

## Claims

1. A surgical cutting tool (10) for use with a handpiece (12) having a motor, the surgical cutting tool (10) comprising:
an outer tube (16) extending longitudinally between proximal and distal ends (18, 20) and comprising an inner surface (22) defining a lumen (24) therethrough;
a flexible driveshaft (26) comprising an inner tube (28) rotatably disposed in said lumen (24) and extending in a longitudinal direction (X) past said distal end (20) to a cutting end (30) outside of said lumen (24), said driveshaft (26) further comprising:
at least two torsion sections (36) spaced from one another longitudinally along said inner tube (28) and each a having at least one channel (38) extending through said inner tube (28); and
a bearing section (40) disposed longitudinally between said torsion sections (36),
wherein said channels (38) are configured to collapse under rotational load in response to transmission of torque as said driveshaft (26) rotates such that said torsion sections (36) are spaced apart from said inner surface (22) of said outer tube (16) to a greater extent than said bearing section (40) for reducing friction between said inner tube (28) and said outer tube (16).

2. The surgical cutting tool (10) as set forth in claim 1, wherein each of said torsion and bearing sections (40) has an outer diameter, with said outer diameter of said torsion sections (36) configured to reduce at least five percent when said channels (38) collapse, wherein preferably said outer diameters of said torsion and bearing sections (40) are substantially equal when said driveshaft (26) is stationary.

3. The surgical cutting tool (10) as set forth in any one of the preceding claims, wherein said outer tube (16) and said lumen (24) are disposed in a non-linear configuration between said ends, and said driveshaft (26) is correspondingly disposed in the non-linear configuration within said lumen (24).

4. The surgical cutting tool (10) as set forth in any one of the preceding claims, wherein said inner tube (28) comprises a pair of channel walls (42) individually corresponding to and defining each of said at least one channel (38), wherein preferably said pair of channel walls (42) oppose and face toward one another to define a width (W) of said channel (38) therebetween wherein more preferably said width (W) between said pair of channel walls (42) is at least 15 microns.

5. The surgical cutting tool (10) as set forth in claim 4, wherein said pair of channel walls (42) extend substantially parallel to one another, with said pair of channel walls (42) configured to draw toward one another and collapse said channel (38) when under rotational load in response to transmission of torque as said driveshaft (26) rotates.

6. The surgical cutting tool (10) as set forth in any one of the preceding claims, wherein said at least one channel (38) extends in a helical configuration longitudinally along and about said inner tube (28), wherein preferably said at least one channel (38) extends about said inner tube (28) less than 360 degrees, more preferably less than 180 degrees.

7. The surgical cutting tool (10) as set forth in claim 6, wherein said at least one channel (38) in said helical configuration extends at an angle at or between 30 and 60 degrees from an axis (O) orthogonal to the longitudinal direction (X) of the inner tube (28).

8. The surgical cutting tool (10) as set forth in any one of the preceding claims, wherein said at least one channel (38) comprises a plurality of channels (38) evenly spaced from one another wherein preferably said plurality of channels (38) have a uniform size and shape.

9. The surgical cutting tool (10) as set forth in any one of the preceding claims, wherein each of said torsion and bearing sections (40) has a length along said longitudinal direction (X) of said inner tube (28), with said length of said torsion sections (36) having a ratio of at least 2:1 relative to said length of said bearing section (40).

10. The surgical cutting tool (10) as set forth in claim 1,
wherein said at least one channel (38) of each torsion section (36) comprises a plurality of channels (38),
wherein said plurality of channels (38) extend in a helical configuration longitudinally along and about said inner tube (28) in a first rotational direction (R1); and
wherein said driveshaft (26) is configured to rotate in a second rotational direction (R2), opposite said first rotational direction (R1), and said channels (38) are configured to collapse under rotational load in response to transmission of torque as said driveshaft (26) rotates in said second rotational direction (R2) such that said outer diameter of said torsion section (36) is reduced.

11. The surgical cutting tool (10) as set forth in claim 10, wherein each of said plurality of channels (38) extends about said inner tube (28) less than 360 degrees.

12. The surgical cutting tool (10) as set forth in claim 10, wherein each of said plurality of channels (38) extends about said inner tube (28) less than 180 degrees.

13. The surgical cutting tool (10) as set forth in any one of claims 10-12, wherein each of said plurality of channels (38) in said helical configuration extends at an angle at or between 30 and 60 degrees from an axis (O) orthogonal to the longitudinal direction (X) of the inner tube (28).

14. The surgical cutting tool (10) as set forth in any one of claims 10-13, wherein said outer diameter of said torsion section (36) is reduced by at least five percent.

## Patentansprüche

1. Chirurgisches Schneidwerkzeug (10) zur Verwendung mit einem Handstück (12) mit einem Motor, wobei das chirurgische Schneidwerkzeug (10) Folgendes umfasst:
ein Außenrohr (16), das sich in Längsrichtung zwischen proximalen und distalen Enden (18, 20) erstreckt und eine Innenfläche (22) umfasst, die ein dadurch verlaufendes Lumen (24) definiert;
eine flexible Antriebswelle (26), die ein Innenrohr (28) umfasst, das drehbar in dem Lumen (24) angeordnet ist und sich in einer Längsrichtung (X) über das distale Ende (20) hinaus zu einem Schneidende (30) außerhalb des Lumens (24) erstreckt, wobei die Antriebswelle (26) ferner Folgendes umfasst:
mindestens zwei Torsionsabschnitte (36), die in Längsrichtung entlang des Innenrohrs (28) voneinander beabstandet sind und jeweils mindestens einen Kanal (38) aufweisen, der sich durch das Innenrohr (28) erstreckt; und
einen Lagerabschnitt (40), der in Längsrichtung zwischen den Torsionsabschnitten (36) angeordnet ist,
wobei die Kanäle (38) so konfiguriert sind, dass sie unter Drehbelastung in Reaktion auf die Übertragung von Drehmoment kollabieren, wenn sich die Antriebswelle (26) dreht, so dass die Torsionsabschnitte (36) in einem größeren Ausmaß von der Innenfläche (22) des Außenrohrs (16) beabstandet sind als der Lagerabschnitt (40), um Reibung zwischen dem Innenrohr (28) und dem Außenrohr (16) zu verringern.

2. Chirurgisches Schneidwerkzeug (10) nach Anspruch 1, wobei jeder der Torsions- und Lagerabschnitte (40) einen Außendurchmesser aufweist, wobei der Außendurchmesser der Torsionsabschnitte (36) so konfiguriert ist, dass er sich um mindestens fünf Prozent verringert, wenn die Kanäle (38) kollabieren, wobei vorzugsweise die Außendurchmesser der Torsions- und Lagerabschnitte (40) im Wesentlichen gleich sind, wenn die Antriebswelle (26) ruht.

3. Chirurgisches Schneidwerkzeug (10) nach einem der vorstehenden Ansprüche, wobei das Außenrohr (16) und das Lumen (24) in einer nichtlinearen Konfiguration zwischen den Enden angeordnet sind und die Antriebswelle (26) in der nichtlinearen Konfiguration entsprechend innerhalb des Lumens (24) angeordnet ist.

4. Chirurgisches Schneidwerkzeug (10) nach einem der vorstehenden Ansprüche, wobei das Innenrohr (28) ein Paar Kanalwände (42) umfasst, die jeweils einzeln jedem des mindestens einen Kanals (38) entsprechen und diesen definieren, wobei vorzugsweise die Kanalwände (42) einander gegenüberliegen und einander zugewandt sind, um eine Breite (W) des Kanals (38) dazwischen zu definieren, wobei besonders bevorzugt die Breite (W) zwischen dem Paar Kanalwände (42) mindestens 15 Mikrometer beträgt.

5. Chirurgisches Schneidwerkzeug (10) nach Anspruch 4, wobei sich das Paar Kanalwände (42) im Wesentlichen parallel zueinander erstreckt, wobei das Paar Kanalwände (42) so konfiguriert ist, dass es sich unter einer Rotationsbelastung als Reaktion auf die Übertragung eines Drehmoments beim Drehen der Antriebswelle (26) aufeinander zu bewegt und den Kanal (38) kollabiert.

6. Chirurgisches Schneidwerkzeug (10) nach einem der vorstehenden Ansprüche, wobei sich der mindestens eine Kanal (38) in einer spiralförmigen Konfiguration in Längsrichtung entlang und um das Innenrohr (28) erstreckt, wobei sich vorzugsweise der mindestens eine Kanal (38) über weniger als 360 Grad, besonders bevorzugt über weniger als 180 Grad um das Innenrohr (28) erstreckt.

7. Chirurgisches Schneidwerkzeug (10) nach Anspruch 6, wobei sich der mindestens eine Kanal (38) in der spiralförmigen Konfiguration in einem Winkel von oder zwischen 30 bis 60 Grad von einer Achse (O) erstreckt, die orthogonal zur Längsrichtung (X) des Innenrohrs (28) verläuft.

8. Chirurgisches Schneidwerkzeug (10) nach einem der vorstehenden Ansprüche, wobei der mindestens eine Kanal (38) eine Vielzahl von Kanälen (38) umfasst, die gleichmäßig voneinander beabstandet sind, wobei vorzugsweise die Vielzahl von Kanälen (38) eine einheitliche Größe und Form aufweist.

9. Chirurgisches Schneidwerkzeug (10) nach einem der vorstehenden Ansprüche, wobei jeder der Torsions- und Lagerabschnitte (40) eine Länge entlang der Längsrichtung (X) des Innenrohrs (28) aufweist, wobei die Länge der Torsionsabschnitte (36) ein Verhältnis von mindestens 2:1 in Bezug auf die Länge des Lagerabschnitts (40) aufweist.

10. Chirurgisches Schneidwerkzeug (10) nach Anspruch 1,
wobei der mindestens eine Kanal (38) jedes Torsionsabschnitts (36) mehrere Kanäle (38) umfasst,
wobei sich die mehreren Kanäle (38) in einer spiralförmigen Konfiguration in Längsrichtung entlang und um das Innenrohr (28) in einer ersten Drehrichtung (R1) erstrecken; und
wobei die Antriebswelle (26) so konfiguriert ist, dass sie sich in einer zweiten Drehrichtung (R2) entgegengesetzt zur ersten Drehrichtung (R1) dreht, und die Kanäle (38) so konfiguriert sind, dass sie unter Drehbelastung in Reaktion auf die Übertragung von Drehmoment kollabieren, wenn sich die Antriebswelle (26) in der zweiten Drehrichtung (R2) dreht, so dass der Außendurchmesser des Torsionsabschnitts (36) verringert wird.

11. Chirurgisches Schneidwerkzeug (10) nach Anspruch 10, wobei jeder der mehreren Kanäle (38) sich über weniger als 360 Grad um das Innenrohr (28) erstreckt.

12. Chirurgisches Schneidwerkzeug (10) nach Anspruch 10, wobei jeder der mehreren Kanäle (38) sich über weniger als 180 Grad um das Innenrohr (28) erstreckt.

13. Chirurgisches Schneidwerkzeug (10) nach einem der Ansprüche 10-12, wobei sich jeder der mehreren Kanäle (38) in der spiralförmigen Konfiguration in einem Winkel von oder zwischen 30 bis 60 Grad zu einer Achse (O) erstreckt, die orthogonal zur Längsrichtung (X) des Innenrohrs (28) verläuft.

14. Chirurgisches Schneidwerkzeug (10) nach einem der Ansprüche 10-13, wobei der Außendurchmesser des Torsionsabschnitts (36) um mindestens fünf Prozent reduziert ist.

## Revendications

1. Outil coupant chirurgical (10) destiné à être utilisé avec une pièce à main (12) ayant un moteur, l'outil coupant chirurgical (10) comprenant:
un tube extérieur (16) s'étendant longitudinalement entre les extrémités proximale et distale (18, 20) et comprenant une surface intérieure (22) y délimitant une lumière (24);
un arbre d'entraînement flexible (26) comprenant un tube intérieur (28) disposé de manière rotative dans ladite lumière (24) et s'étendant dans une direction longitudinale (X) au-delà de ladite extrémité distale (20) jusqu'à une extrémité coupante (30) à l'extérieur de ladite lumière (24), ledit arbre d'entraînement (26) comprenant en outre:
au moins deux sections de torsion (36) espacées l'une de l'autre longitudinalement le long dudit tube intérieur (28) et comportant chacune au moins un canal (38) traversant ledit tube intérieur (28); et
une section de roulement (40) disposée longitudinalement entre lesdites sections de torsion (36),
dans lequel lesdits canaux (38) sont conçus pour s'affaisser sous la charge de rotation en réponse à la transmission du couple lorsque ledit arbre d'entraînement (26) tourne de telle sorte que lesdites sections de torsion (36) sont espacées de ladite surface intérieure (22) dudit tube extérieur (16) dans une plus grande mesure que ladite section de roulement (40) pour réduire le frottement entre ledit tube intérieur (28) et ledit tube extérieur (16).

2. Outil coupant chirurgical (10) selon la revendication 1, dans lequel chacune desdites sections de torsion et de roulement (40) a un diamètre extérieur, ledit diamètre extérieur desdites sections de torsion (36) étant conçu pour se réduire d'au moins cinq pour cent lorsque lesdits canaux (38) s'affaissent, lesdits diamètres extérieurs desdites sections de torsion et de roulement (40) étant de préférence sensiblement égaux lorsque ledit arbre d'entraînement (26) est stationnaire.

3. Outil coupant chirurgical (10) selon l'une quelconque des revendications précédentes, dans lequel ledit tube extérieur (16) et ladite lumière (24) sont disposés dans une configuration non linéaire entre lesdites extrémités, et ledit arbre d'entraînement (26) est disposé de manière correspondante dans la configuration non linéaire à l'intérieur de ladite lumière (24).

4. Outil coupant chirurgical (10) selon l'une quelconque des revendications précédentes, dans lequel ledit tube intérieur (28) comprend une paire de parois de canal (42) correspondant individuellement à chacun dudit au moins un canal (38) et définissant ce dernier, dans lequel de préférence ladite paire de parois de canal (42) sont en regard et se font face l'une de l'autre pour définir une largeur (W) dudit canal (38) entre elles, dans lequel plus préférablement ladite largeur (W) entre ladite paire de parois de canal (42) est d'au moins 15 microns.

5. Outil coupant chirurgical (10) selon la revendication 4, dans lequel ladite paire de parois de canal (42) s'étend sensiblement parallèle l'une à l'autre, ladite paire de parois de canal (42) étant conçue pour se rapprocher l'une de l'autre et pour affaisser ledit canal (38) lorsqu'il est soumis à une charge de rotation en réponse à la transmission du couple lors de la rotation de l'arbre d'entraînement (26).

6. Outil coupant chirurgical (10) selon l'une quelconque des revendications précédentes, dans lequel ledit au moins un canal (38) s'étend dans une configuration hélicoïdale longitudinalement le long dudit tube intérieur (28) et autour de ce dernier, dans lequel de préférence ledit au moins un canal (38) s'étend autour dudit tube intérieur (28) sur moins de 360 degrés, plus préférentiellement sur moins de 180 degrés.

7. Outil coupant chirurgical (10) selon la revendication 6, dans lequel ledit au moins un canal (38) dans ladite configuration hélicoïdale s'étend à un angle à ou entre 30 et 60 degrés par rapport à un axe (O) orthogonal à la direction longitudinale (X) du tube intérieur (28).

8. Outil coupant chirurgical (10) selon l'une quelconque des revendications précédentes, dans lequel ledit au moins un canal (38) comprend une pluralité de canaux (38) espacés à intervalles réguliers les uns des autres, dans lequel, de préférence, ladite pluralité de canaux (38) a une taille et une forme uniformes.

9. Outil coupant chirurgical (10) selon l'une quelconque des revendications précédentes, dans lequel chacune desdites sections de torsion et de roulement (40) a une longueur le long de ladite direction longitudinale (X) dudit tube intérieur (28), ladite longueur desdites sections de torsion (36) ayant un rapport d'au moins 2:1 par rapport à ladite longueur de ladite section de roulement (40).

10. Outil coupant chirurgical (10) selon la revendication 1,
dans lequel ledit au moins un canal (38) de chaque section de torsion (36) comprend une pluralité de canaux (38),
dans lequel ladite pluralité de canaux (38) s'étend dans une configuration hélicoïdale longitudinalement le long et autour dudit tube intérieur (28) dans une première direction de rotation (R1); et
dans lequel ledit arbre d'entraînement (26) est conçu pour tourner dans un second sens de rotation (R2), opposé au premier sens de rotation (R1), et lesdits canaux (38) sont conçus pour s'affaisser sous la charge de rotation en réponse à la transmission du couple lorsque ledit arbre d'entraînement (26) tourne dans ladite seconde direction de rotation (R2) de sorte que ledit diamètre extérieur de ladite section de torsion (36) est réduit.

11. Outil coupant chirurgical (10) selon la revendication 10, dans lequel chacun de la pluralité de canaux (38) s'étend autour du tube intérieur (28) sur moins de 360 degrés.

12. Outil coupant chirurgical (10) selon la revendication 10, dans lequel chacun de la pluralité de canaux (38) s'étend autour du tube intérieur (28) sur moins de 180 degrés.

13. Outil coupant chirurgical (10)selon l'une quelconque des revendications 10 à 12, dans lequel chacun de la pluralité de canaux (38) dans cette configuration hélicoïdale s'étend à un angle à ou entre 30 et 60 degrés par rapport à un axe (O) orthogonal à la direction longitudinale (X) du tube intérieur (28).

14. Outil coupant chirurgical (10) selon l'une quelconque des revendications 10 à 13, dans lequel le diamètre extérieur de la section de torsion (36) est réduit d'au moins cinq pour cent.
